(19) Europäisches Patentamt
European Patent Office
Office européen des brevets

(11) Veröffentlichungsnummer: **0 224 723 B1**

(12) **EUROPÄISCHE PATENTSCHRIFT**

(45) Veröffentlichungstag der Patentschrift: **23.11.89**

(51) Int. Cl.⁴: **C07D 233/60**, C07D 249/08, C07D 231/10, C07F 7/10

(21) Anmeldenummer: **86115109.0**

(22) Anmeldetag: **31.10.86**

(54) **Verfahren zur Herstellung von Beta-Hydroxyethyl-azol-Derivaten.**

(30) Priorität: **12.11.85 DE 3539993**

(43) Veröffentlichungstag der Anmeldung: **10.06.87 Patentblatt 87/24**

(45) Bekanntmachung des Hinweises auf die Patenterteilung: **23.11.89 Patentblatt 89/47**

(84) Benannte Vertragsstaaten: **BE CH DE FR GB IT LI NL**

(56) Entgegenhaltungen:
**EP-A- 0 068 813**
**EP-A- 0 143 379**

**HOUBEN-WEYL "Methoden der Organischen Chemie", Band VI/1a, Teil 2, 1980, Seite 928, Georg Thieme Verlag, Stuttgart; H. KROPF et al.: "Herstellung von Alkoholen durch Aufbaureaktionen"**
**TETRAHEDRON LETTERS, Band 23, Nr. 48, 1982, Seiten 5079-5082, Oxford, GB; A. RICCI et al.: "Fluoride ion induced reactions of organosilanes with saturated lactones and alpha beta-enones"**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Lantzsch, Reinhard, Dr., Am Buschhäuschen 51, D-5600 Wuppertal 1(DE)**
Erfinder: **Kranz, Eckard, Dr., Am Acker 9, D-5600 Wuppertal 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 224 723 B1**

ACTORUM AG

## Beschreibung

Die vorliegende Erfindung betrifft ein neues Verfahren zur Herstellung von bekannten β-Hydroxyethyl-azol-Derivaten, welche fungizide und pflanzenwuchsregulierende Eigenschaften besitzen. Die Erfindung betrifft außerdem ein neues Zwischenprodukt, das zur Herstellung von β-Hydroxyethyl-azol-Derivaten verwendet werden kann.

Es ist bereits bekannt geworden, daß man β-Hydroxyethyl-azol-Derivate herstellen kann, indem man entsprechende Ketone entweder

– mit Dimethyloxosulfonium-methylid in Gegenwart eines Verdünnungsmittels, wie z.B. Dimethylsulfoxid, bei Temperaturen zwischen 20 und 80°C umsetzt (vgl. J. Am. Chem. Soc. 87, 1363–1364 (1965) oder

– mit Trimethylsulfonium-methylsulfat in Gegenwart eines inerten organischen Verdünnungsmittels, wie z.B. Acetonitril, und in Gegenwart einer Base, wie z.B. Natrium-methylat, bei Temperaturen zwischen 0°C und 60°C umsetzt (vgl. Heterocycles 8, 397 (1977), und

anschließend die jeweils entstehenden Oxiran-Derivate mit Azolen in Gegenwart einer Base, wie z.B. Natriumethylat, und in Gegenwart eines inerten organischen Lösungsmittels, wie beispielsweise Ethanol, bei Temperaturen zwischen 60 und 150 °C umsetzt (vgl. EP-A 40 345, EP-A 40 605, EP-A 44 337, EP-A 46 337, EP-A 47 594, EP-A 52 424 und DE-A 2 654 890).

Nachteilig an diesen Verfahren ist jedoch, daß die in der ersten Stufe erfolgende Epoxidierung der Ketone in technischem Maßstab oft nur schwierig durchführbar ist, da wasserfreie Lösungsmittel verwendet werden müssen. Außerdem erfordert diese Reaktion unter Umständen den Einsatz teurer Basen, wie beispielsweise Kalium-tert.-butylat. Zusätzlich können Abluftprobleme aufgrund des niedrigen Siedepunkts des entstehenden Dimethylsulfids (37°C) auftreten.

Bei der in der zweiten Stufe erfolgenden Öffnung der Epoxide mit Azol tritt bei der Verwendung von 1,2,4-Triazol ein weiterer Nachteil auf, da sich außer den 1,2,4-Triazol-Derivaten je nach dem eingesetzten Solvens mehr oder weniger große Mengen an 1,3,4-Triazol-Derivaten bilden. Arbeitet man beispielsweise in Gegenwart von Alkoholen, so entstehen bis zu 30% an den unerwünschten Stoffen. Die Abtrennung dieser störenden Nebenprodukte ist aufwendig und die Ausbeuten an den gewünschten 1,2,4-Triazol-Derivaten liegen daher relativ niedrig.

Wie aus den Publikationen in Houben-Weyl, Band IV/la, Teil 2, 1980, Seiten 1482–1483 und in Tetrahedron Letters 23, 5079–5082 (1982) hervorgeht, lassen sich bestimmte Trimethylsilyl-Verbindungen unter der katalytischen Einwirkung von Fluorid-Ionen mit Elektrophilen, wie zum Beispiel Carbonyl-Gruppen, so umsetzen, daß der mit dem Silizium-Atom verbundene Rest R an die Carbonyl-Gruppe addiert wird. In allen Fällen handelt es sich bei dem Substituenten R um einen Rest, in dem das reagierende, dem Silizium-Atom benachbarte Kohlenstoffatom mit einem weiteren Kohlenstoffatom verknüpft ist. Entsprechende Reaktionen von Verbindungen, in denen das Kohlenstoffatom in Nachbarstellung zum Silizium-Atom an ein Heteroatom eines heterocyclischen Ringes gebunden ist, werden jedoch in keiner der beiden oben zitierten Druckschriften offenbart.

Es wurde nun gefunden, daß man die bekannten β-Hydroxy-ethyl-azol-Derivate der Formel

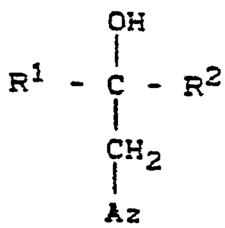

(I)

in welcher

$R^1$ für geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cyclo-alkenyl mit 5 bis 8 Kohlenstoffatomen, oder für gegebenenfalls ein- bis dreifach, gleichartig oder verschieden substituiertes Phenyl steht, wobei als Substituenten in Frage kommen: Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil oder

$R^1$ für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, und

$R^2$ für Wasserstoff, geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cyclo-alkenyl mit 5 bis 8 Kohlenstoffatomen steht, oder für gegebenenfalls ein- bis dreifach, gleichartig oder verschieden substituiertes Phenyl steht, wobei als Substituenten in Frage kommen: Halogen, geradkettiges oder verzweigtes Alkyl mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogentatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und gegebenenfalls durch Halogen und/oder Alkyl mit 1 bis 4 Kohlenstoffatomen substituiertes Phenylalkoxy mit 1 oder 2 Kohlenstoffatomen im Alkoxyteil, und

$R^2$ außerdem für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil und Az für 1,2,4-Triazol-1-yl, Imidazol-1-yl und Pyrazol-1-yl steht, erhält, wenn man Carbonyl-Verbindungen der Formel

$R^1\!-\!CO\!-\!R^2$

in welcher

$R^1$ und $R^2$ die oben angegebene Bedeutung haben, mit Trimethylsilyl-azolyl-methanen der Formel

$(CH_3)_3\,Si\!-\!CH_2\!-\!Az$ (III)

in welcher

Az die oben angegebene Bedeutung hat, in Gegenwart eines Katalysators und gegebenenfalls in Gegenwart eines Verdünnungsmittels bei Temperaturen zwischen – 20°C und +130°C umsetzt.

Es ist als äußerst überraschend zu bezeichnen, daß das erfindungsgemäße Verfahren so breit anwendbar ist. Beim Einsatz von Ketonen der Formel (II) war lediglich eine Silylierung der Enolform zu erwarten, wie dies von der folgenden Reaktion bekannt ist:

$$R' - CO - CH_2 - R'' + (CH_3)_3Si - CH_2 - COOC_2H_5 \longrightarrow$$

$$R' - \underset{\displaystyle |\atop \displaystyle OSi(CH_3)_3}{C} = CH - R'' + CH_3 - COOC_2H_5$$

(vgl. Tetrahedron Letters 1978, 2079 und J. Am. Chem. Soc. 98, 2346 (1976).

Besonders überraschend ist, daß der Katalysator beim erfindungsgemäßen Verfahren auch in seiner Hydrat-Form eingesetzt werden kann, da in Gegenwart von Wasser folgende Reaktionen zu erwarten wären:

$$(CH_3)_3Si - CH_2 - Az \xrightarrow{+ H_2O} (CH_3)_3SiOH + CH_3 - Az$$

$$2\ (CH_3)_3SiOH \xrightarrow{+ H_2O} (CH_3)_3Si - O - Si(CH_3)_3$$

Das erfindungsgemäße Verfahren zeichnet sich durch eine Reihe von Vorteilen aus. So ermöglicht es die Herstellung von β-Hydroxyethyl-azol-Derivaten der Formel (I) in hohen Ausbeuten, wobei billige und gut zugängliche Verbindungen als Ausgangsprodukte eingesetzt werden. Ferner ist die Umsetzung einfach durchführbar, und die Isolierung der Verbindungen der Formel (I) bereitet keinerlei Schwierigkeiten.

Verwendet man beispielsweise 1-(4-Chlorphenyl)-4,4-dimethyl-pentan-3-on und Trimethylsilyl-(1,2,4-triazol-1-yl)methan als Ausgangsstoffe und wäßrige Tetrabutyl-ammoniumfluorid-Lösung als Katalysator, so kann der Verlauf des erfindungsgemäßen Verfahrens durch das folgende Formelschema veranschaulicht werden:

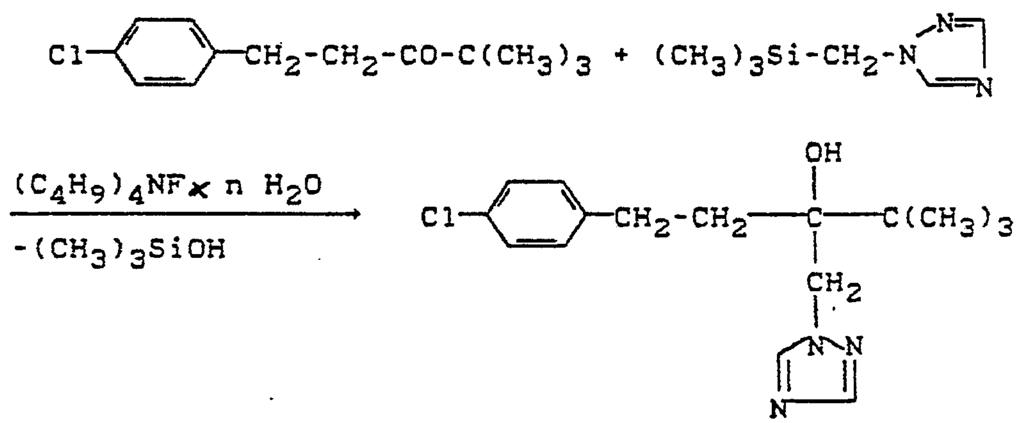


Die bei dem erfindungsgemäßen Verfahren als Ausgangsstoffe zu verwendenden Keto-Derivate sind durch die Formel (II) allgemein definiert.

Bevorzugte Ausgangsstoffe sind diejenigen Verbindungen der Formel (II), in denen

$R^1$ für Methyl, Ethyl, Propyl, Isopropyl, n-Butyl, iso-Butyl, sek.-Butyl, tert.-Butyl, durch 1 bis 3 Fluor- und/oder Chloratome substituiertes Methyl, Ethyl, Propyl, Isopropyl, n-Butyl, iso-Butyl, sek.-Butyl oder tert.-Butyl steht, ferner für Alkenyl mit 2 bis 6 Kohlenstoffatomen, Alkinyl mit 2 bis 6 Kohlenstoffatomen, gegebenenfalls durch Methyl substituiertes Cyclopropyl, Cyclopentyl oder Cyclohexyl, gegebenenfalls durch Methyl substituiertes Cyclo-pentenyl, Cyclohexenyl, Cycloheptenyl oder Cyclo-octenyl steht, weiterhin für Phenyl steht, welches 1 bis dreifach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy, und $R^1$ außerdem für Phenethyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy;

$R^2$ für Wasserstoff, Methyl, Ethyl, Propyl, Isopropyl, n-Butyl, iso-Butyl, sek.-Butyl, tert.-Butyl, durch 1 bis 3 Fluor- und/oder Chloratome substituiertes Methyl, Ethyl, Propyl, Isopropyl, n-Butyl, iso-Butyl, sek.-Butyl oder tert.-Butyl steht, ferner für Alkenyl mit 2 bis 6 Kohlenstoffatomen, gegebenenfalls durch Methyl substituiertes Cyclopropyl, Cyclopentyl oder Cyclohexyl, gegebenenfalls durch Methyl substituiertes Cyclopentenyl, Cyclohexenyl, Cycloheptenyl oder Cyclooctenyl steht, weiterhin für Phenyl steht, welches 1 bis dreifach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy, und

$R^2$ außerdem für Phenethyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy.

Die Carbonyl-Verbindungen der Formel (II) sind allgemein bekannte Verbindungen der organischen Chemie.

Die für das erfindungsgemäße Verfahren außerdem als Ausgangsstoffe zu verwendenden Trimethylsilylazolylmethane sind durch die Formel (III) allgemein definiert. In dieser Formel steht Az vorzugsweise für die in der Erfindungsdefinition angegebenen Bedeutungen.

Die Trimethylsilyl-azolyl-methane der Formel (III) sind teilweise bekannt (vgl. US-A 3 692 798, EP-A 68 813 sowie J. Org. Chem. 49, 4687–95). Noch nicht beschrieben ist das Trimethylsilyl-(1,2,4-triazol-1-yl)-methan der Formel

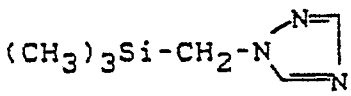

(III-1).

Man erhält das Trimethylsilyl-(1,2,4-triazol-1-yl)-methan der Formel (III-1), indem man Trimethyl-chlormethyl-silan der Formel

$(CH_3)_3SiCH_2Cl$ (IV)

mit 1,2,4-Triazol der Formel

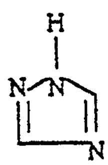

(V)

in Gegenwart eines Säurebindemittels und in Gegenwart eines Verdünnungsmittels umsetzt.

Trimethyl-chlormethyl-silan der Formel (IV) und 1,2,4-Triazol der Formel (V) sind bekannt.

Bei der Herstellung von Trimethylsilyl-(1,2,4-triazol-1-yl)-methan nach dem obigen Verfahren können als Säurebindemittel alle üblichen Säureakzeptoren eingesetzt werden. Vorzugsweise verwendbar sind Alkalimetallcarbonate, wie Natriumcarbonat oder Kaliumcarbonat.

Als Verdünnungsmittel können bei der Herstellung von Trimethylsilyl-(1,2,4-triazol-1-yl)-methan nach dem obigen Verfahren alle üblichen inerten organischen Solventien eingesetzt werden. Vorzugsweise verwendbar sind Ketone, wie z.B. Aceton.

Die Reaktionstemperaturen können bei dem oben genannten Verfahren zur Herstellung von Trimethylsilyl-(1,2,4-triazol-1-yl)-methan innerhalb eines größeren Bereiches variiert werden. Im allgemeinen arbeitet man bei Temperaturen zwischen 20 und 150°C, vorzugsweise zwischen 40 und 120°C.

Bei der Durchführung des obigen Verfahrens zur Herstellung von Trimethylsilyl-(1,2,4-triazol-1-yl)-methan setzt man die Reaktionskomponenten im allgemeinen in äquivalenten Mengen ein. Es ist jedoch auch möglich, die eine oder die andere Komponente in einem Überschuß zu verwenden. Die Aufarbeitung erfolgt nach üblichen Methoden (vgl. Herstellungsbeispiele).

Als Katalysatoren kommen für die erfindungsgemäße Umsetzung vorzugsweise Tetraalkylammoniumfluoride infrage, wie beispielsweise Tetraethylammoniumfluorid oder Tetrabutylammoniumfluorid. Hervorzuheben ist, daß diese Fluoride auch in Form ihrer Hydrate eingesetzt werden.

Als Verdünnungsmittel kommen bei der Durchführung des erfindungsgemäßen Verfahrens alle unter den Reaktionsbedingungen inerten organischen Solventien in Betracht.

Vorzugsweise verwendbar sind Ether, wie Diethylether, Diisopropylether, 1,2-Dimethoxymethan, Tetrahydrofuran oder Dioxan; aromatische Kohlenwasserstoffe, wie Toluol oder Chlorbenzol; chlorierte Kohlenwasserstoffe, wie Methylenchlorid oder Chloroform; ferner Acetonitril, Dimethylformamid, Dimethylsulfoxid und N-Methylpyrrolidon.

Die Reaktionstemperaturen können bei der Durchführung des erfindungsgemäßen Verfahrens in einem größeren Bereich variiert werden. Man arbeitet bei Temperaturen zwischen –20 und +130°C, vorzugsweise zwischen 0 und 80°C.

Bei der Durchführung des erfindungsgemäßen Verfahrens setzt man auf 1 Mol Carbonyl-Verbindung der Formel (II) im allgemeinen 1 bis 6 Mol, vorzugsweise 1 bis 4 Mol an Trimethylsilyl-azolyl-methan der Formel (III) ein. Die Menge an Katalysator beträgt im allgemeinen 1 bis 30 Mol-%. Die Isolierung der Endprodukte der Formel (I) erfolgt in üblicher Weise.

In manchen Fällen ist es möglich, die auftretenden Zwischenprodukte der Formel (Ia)

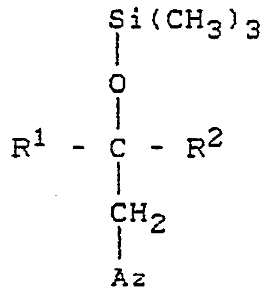

(Ia)

in welcher
$R^1$, $R^2$ und Az die oben angegebene Bedeutung haben, zu isolieren. Entscheidend hierfür ist die Hydrolyse-Stabilität der Verbindungen der Formel (Ia).

Die nach dem erfindungsgemäßen Verfahren herstellbaren β-Hydroxyethyl-azol-Derivate der Formel (I) sind bekannt (vgl. EP-A 40 345, EP-A 44 605, EP-A 46 337, EP-A 47 594; EP-A 52 424, DE-A 2 431 407, DE-A 2 653 420, DE-A 2 640 823, DE-A 2 654 890, DE-A 2 708 987, DE-A 2 735 872 oder US-A 3 303 200). Sie zeichnen sich durch sehr gute fungizide und pflanzenwuchsregulierende Eigenschaften aus.

Das erfindungsgemäße Verfahren wird durch die nachfolgenden Beispiele veranschaulicht.

Beispiel 1

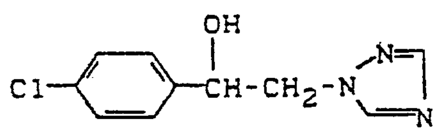

(I-1)

3 g (0,02 Mol) p-Chlorbenzaldehyd und 6,2 g (0,04 Mol) Trimethylsilyl-(1,2,4-triazol-1-yl)-methan werden in 25 ml Tetrahydrofuran gelöst und bei 0°C tropfenweise mit 4 ml 1-molarer Tetrabutylammoniumfluoridlösung in Tetrahydrofuran versetzt. Das Reaktionsgemisch wird 12 Stunden bei 20 bis 25°C gerührt und anschließend durch Abdestillieren des Tetrahydrofurans eingeengt. Der Rückstand wird im Kugelrohr bei 140°C im Hochvakuum (0,1 mbar) destilliert. Man erhält 2,8 g (63% der Theorie) 1-(4-Chlorphenyl)-2-(1,2,4-triazol-1-yl)-1-ethanol vom Schmelzpunkt 116–118°C.

Beispiel 2

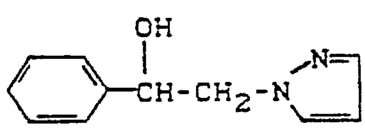

(I-2)

In eine Mischung aus 23,1 g (0,15 Mol) Trimethylsilyl-1-pyrazolyl-methan und 7,95 g (0,075 Mol) Benzaldehyd gibt man portionsweise 4,7 g (0,015 Mol) Tetrabutylammonium-fluorid (Trihydrat). Dann erhitzt man vorsichtig, bis eine exotherme Reaktion einsetzt. Durch Kühlung hält man das Reaktionsgemisch bei 80°C und rührt dann noch eine Stunde bei dieser Temperatur nach. Nach dem Abkühlen versetzt man mit 150 ml Wasser, 2 Tropfen Salzsäure und 150 ml Methylenchlorid, trennt die Methylenchloridphase ab und engt ein. Nach Verreiben des Rückstandes mit Petrolether erhält man 6,8 g (47,6% der Theorie) 1-Phenyl-2-(1-pyrazolyl)-1-ethanol vom Schmelzpunkt 125–128°C.

Beispiel 3

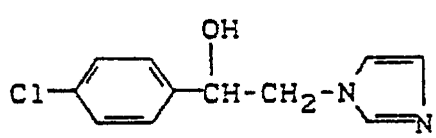

(I-3)

Zu einer Mischung aus 23,1 g (0,15 Mol) Trimethylsilyl-1-imidazolyl-methan und 7,95 g (0,075 Mol) Benzaldehyd gibt man 4,7 g (0,015 Mol) Tetrabutylammoniumfluorid (Trihydrat). Die Reaktion verläuft stark exotherm. Nach dem Abkühlen versetzt man mit 150 ml Methylenchlorid und 150 ml Wasser, trennt die organische Phase ab, trocknet und engt ein. Nach dem Abpressen auf Ton erhält man 8,1 g (57,8% der Theorie) 1-Phenyl-2-(imidazol-1-yl)-1-ethanol vom Schmelzpunkt 142–145°C.

Beispiel 4

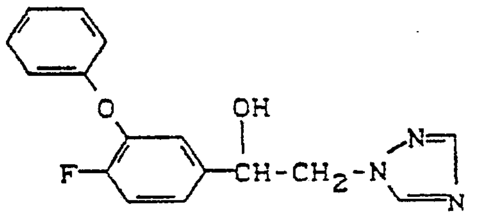

(I-4)

Zu einer Mischung von 10,8 g (0,05 Mol) 4-Fluor-3-phenoxy-benzaldehyd und 15,5 g (1 Mol) Trimethylsilyl-(1,2,4-triazol-1-yl)methan gibt man unter Rühren 3,15 g (0,01 Mol) Tetrabutylammoniumfluorid (Trihydrat). Man hält das Reaktionsgemisch dabei bei 50°C. Anschließend läßt man abkühlen und versetzt mit 50 ml Methylenchlorid sowie mit 50 ml Wasser, dem einige Tropfen verdünnte Salzsäure zugegeben werden. Die organische Phase wird abgetrennt, mehrmals mit Wasser gewaschen, getrocknet und eingeengt. Der Rückstand wird im Kugelrohr bei 250°C im Hochvakuum (0,1 mbar) destilliert. Man erhält 7,7 g (41,5% der Theorie 1-(4-Fluor-3-phenoxy-phenyl)-2-(1,2,4-triazol-1-yl)-1-ethanol vom Schmelzpunkt 78°C.

Beispiel 5

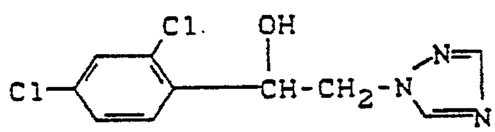

(I-5)

3,56 g (0,02 Mol) frisch destillierter 2,4-Dichlor-benzal-dehyd und 4 ml 1-molare Tetrabutylammoniumfluoridlösung in Tetrahydrofuran werden in 25 ml Tetrahydrofuran zum Sieden erhitzt und tropfenweise mit 6,2 g (0,06 Mol) Trimethylsilyl-(1,2,4-triazol-1-yl)-methan in 5 ml Tetrahydofuran versetzt. Das Reaktionsgemisch wird anschließend 12 Stunden unter Rückfluß erhitzt. Man läßt abkühlen, verdünnt mit Wasser und extrahiert dreimal mit Methylen-chlorid. Die vereinigten organischen Phasen werden getrocknet und eingeengt. Der Rückstand wird im Hochvakuum (0,1 mbar) bis 100°C destilliert. Der Rückstand wird mit Ether verrührt und abfiltriert. Man erhält 2,3 g (44,7% der Theorie) 1-(2,4-Dichlorphenyl)-2,(1,2,4-triazol-1-yl)-1-ethanol vom Schmelzpunkt 90–91°C.

Beispiel 6

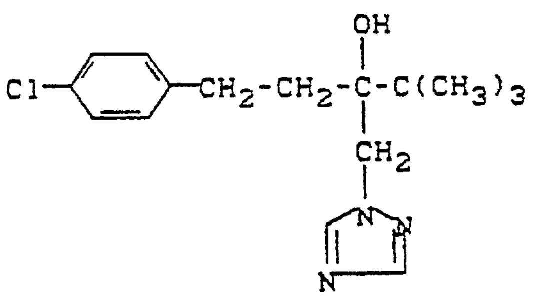

(I-6)

Zu 4,49 g (0,02 Mol) 1-(4-Chlorphenyl)-4,4-dimethyl-pen-tan-3-on und 6,2 g (0,04 Mol) Trimethylsilyl-(1,2,4-tria-zol-1-yl)-methan werden unter Rühren 4 ml 1-molare Tetra-butylammoniumfluoridlösung in Tetrahydrofuran zugetropft. Man erhitzt das Reaktionsgemisch 12 Stunden auf 80°C, läßt danach abkühlen, verdünnt mit Wasser und extrahiert mit Methylenchlorid. Die organische Phase wird getrocknet und eingeengt. Der Rückstand wird mit Benzin verrührt. Man erhält 4,9 g (64,6% der Theorie) 1-(4-Chlorphenyl)-4,4-dimethyl-3-(1,2,4-triazol-1-yl-methyl)-pentan-3-ol vom Schmelzpunkt 104°C.

In analoger Weise werden die in der folgenden Tabelle 1 aufgeführten Produkte erhalten:

Tabelle 1

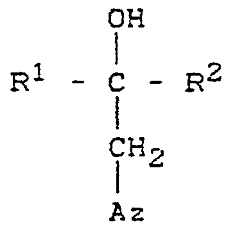

(I)

| Bsp. Nr. | $R^1$ | $R^2$ | Az | Fp (°C) bzw. $n_D^{20}$ |
|---|---|---|---|---|
| I-7 | 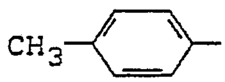 | H | 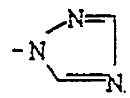 | 130-31 |
| I-8 | 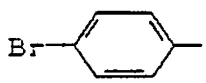 | H | 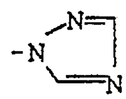 | 120-22 |
| I-9 | 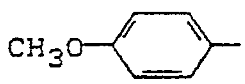 | H | 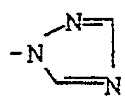 | 103-04 |
| I-10 | $(CH_3)_2C{=}CH-$ | $CH_3$ | 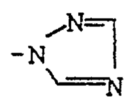 | $n_D^{20}$ = 1,479 |
| I-11 | 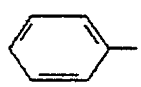 | $CH_3$ | 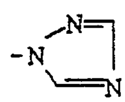 | 28 |
| I-12 | $C_6H_{13}-$ | H | 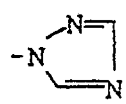 | 40-42 |

Herstellung von Ausgangsprodukten

Beispiel (III-1)

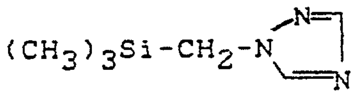 (III-1)

245 g (2 Mol) Trimethyl-chlormethyl-silan in 1 l Aceton und 331 g (2,4 Mol) Kaliumcarbonat werden vorgelegt. Dazu gibt man portionsweise bei Raumtemperatur 138 g (2 Mol) fein gepulvertes 1,2,4-Triazol. Das Reaktionsgemisch wird 30 Stunden unter Rückfluß erhitzt und anschließend abgesaugt, mit Aceton nachgewaschen, eingeengt und im Vakuum destilliert. Man erhält 161,8 g (52% der Theorie) Trimethylsilyl-(1,2,4-triazol-1-yl)-methan vom Siedpunkt 97–99°C/15 mbar.

In analoger Weise werden die in der folgenden Tabelle 2 aufgeführten Ausgangsprodukte erhalten.

| Bsp. Nr. | Az | Kp(°C)/mbar |
|---|---|---|
| III-2 | 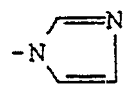 | 80-81/0,7 |
| III-3 | 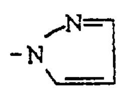 | 66-68/16 |

**Patentansprüche**

1. Verfahren zur Herstellung von β-Hydroxyethylanzol-Derivaten der Formel

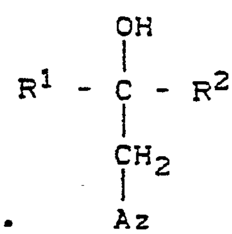 (I)

in welcher

$R^1$ für geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cycloalkenyl mit 5 bis 8 Kohlenstoffatomen, oder für gegebenenfalls ein-bis dreifach, gleichartig oder verschieden substituiertes Phenyl steht, wobei als Substituenten in Frage kommen: Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil oder

$R^1$ für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy

mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil, und

$R^2$ für Wasserstoff, geradkettiges oder verzweigtes Alkyl mit 1 bis 8 Kohlenstoffatomen, geradkettiges oder verzweigtes Halogenalkyl mit 1 bis 4 Kohlenstoffatomen und 1 bis 5 Halogenatomen, Alkenyl mit 2 bis 8 Kohlenstoffatomen, Alkinyl mit 2 bis 8 Kohlenstoffatomen, gegebenenfalls durch Alkyl mit 1 oder 2 Kohlenstoffatomen substituiertes Cycloalkyl mit 3 bis 7 Kohlenstoffatomen, gegebenenfalls durch Alkyl mit 1 oder 2 Kohlenstoffatomen substituiertes Cycloalkenyl mit 5 bis 8 Kohlenstoffatomen steht, oder für gegebenenfalls ein- bis dreifach, gleichartig oder verschieden substituiertes Phenyl steht, wobei als Substituenten in Frage kommen: Halogen, geradkettiges oder verzweigtes Alkyl mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und gegebenenfalls durch Halogen und/oder Alkyl mit 1 bis 4 Kohlenstoffatomen substituiertes Phenylalkoxy mit 1 oder 2 Kohlenstoffatomen im Alkoxyeil, und

$R^2$ außerdem für Phenylalkyl mit 1 bis 4 Kohlenstoffatomen im Alkylteil steht, wobei der Phenylalkyl-Rest im Phenylteil ein- bis dreifach, gleichartig oder verschieden substituiert sein kann durch Halogen, geradkettiges oder verzweigtes Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil und/oder gegebenenfalls durch Halogen und/oder Alkyl mit 1 bis 4 Kohlenstoffatomen substituiertes Phenylalkoxy mit 1 oder 2 Kohlenstoffatomen im Alkoxyteil und

Az für 1,2,4-Triazol-1-yl, Imidazol-1-yl und Pyrazol-1-yl steht, dadurch gekennzeichnet, daß man Carbonyl-Verbindungen der Formel

$R^1$–CO–$R^2$ (II)

in welcher

$R^1$ und $R^2$ die oben angegebene Bedeutung haben, mit Trimethylsilyl-azolyl-methanen der Formel

$(CH_3)_3$ Si–$CH_2$–Az (III)

in welcher Az die oben angegebene Bedeutung hat, in Gegenwart eines Katalysators und gegebenenfalls in Gegenwart eines Verdünnungsmittels bei Temperaturen zwischen –20°C und +130°C umsetzt.

2. Verfahren gemäß Anspruch 1, dadurch gekennzeichnet, daß man als Ausgangsstoffe Carbonyl-Verbindungen der Formel (II) einsetzt, in denen

$R^1$ für Methyl, Ethyl, Propyl, Isopropyl, n-Butyl, iso-Butyl, sek.-Butyl, tert.-Butyl, durch 1 bis 3 Fluor- und/oder Chloratome substituiertes Methyl, Ethyl, Propyl, Isopropyl, n-Butyl, iso-Butyl, sek.-Butyl oder tert.-Butyl steht, ferner für Alkenyl mit 2 bis 6 Kohlenstoffatomen, Alkinyl mit 2 bis 6 Kohlenstoffatomen, gegebenenfalls durch Methyl substituiertes Cyclopropyl, Cyclopentyl oder Cyclohexyl, gegebenenfalls durch Methyl substituiertes Cyclopentenyl, Cyclohexenyl, Cycloheptenyl oder Cyclooctenyl steht, weiterhin für Phenyl steht, welches 1 bis dreifach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy, und $R^1$ außerdem für Phenethyl steht, das im Phenylteil 1 bis 3-fach substituiert sein kann durch Fluor, Chlor, Brom, Methyl, tert.-Butyl, Cyclohexyl, Methoxy, Methylthio, Trifluormethyl, Trifluormethoxy, Trifluormethylthio, gegebenenfalls durch Fluor, Chlor, und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor; Chlor und/oder Methyl substituiertes Benzyloxy; und

$R^2$ für Wasserstoff, Methyl, Ethyl, Propyl, Isopropyl, n-Butyl, iso-Butyl, sek.-Butyl, tert.-Butyl, durch 1 bis 3 Fluor- und/oder Chloratome substituiertes Methyl, Ethyl, Propyl, Isopropyl, n-Butyl, iso-Butyl, sek.-Butyl oder tert.-Butyl steht, ferner für Alkenyl mit 2 bis 6 Kohlenstoffatomen, gegebenenfalls durch Methyl substituiertes Cyclopropyl oder Cyclohexyl, gegebenenfalls durch Methyl substituiertes Cyclopentenyl, Cyclohexenyl, Cycloheptenyl oder Cyclooctenyl steht, weiterhin für Phenyl steht, welches 1 bis dreifach substituiert sein kann durch Fluor, Chlor, Brom, Methyl, tert.-Butyl, Cyclohexyl, Methoxy, Methylthio, Tri-fluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy, und

$R^2$ außerdem für Phenethyl steht, das im Phenylteil 1 bis 3-fach substituiert sein kann durch Fluor, Chlor, Brom, Methyl, tert.-Butyl, Cyclo-hexyl, Methoxy, Methylthio, Trifluormethyl, Trifluormethoxy, Trifluormethylthio, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder durch Fluor, Chlor und/oder Methyl substituiertes Benzyloxy.

3. Verfahren gemäß Anspruch 1, dadurch gekennzeichnet, daß man als Katalysatoren Tetraalkyl-ammonium-fluoride einsetzt.

4. Verfahren gemäß Anspruch 1, dadurch gekennzeichnet, daß man die Umsetzung bei Temperaturen zwischen 0°C und 80°C durchführt.

5. Verfahren gemäß Anspruch 1, dadurch gekennzeichnet, daß man auf 1 Mol Carbonyl-Verbindung der Formel (II) 1 bis 6 Mol an Trimethylsilyl-azolyl-methan der Formel (III) einsetzt.

6. Verfahren gemäß Anspruch 1, dadurch gekennzeichnet, daß man 1-(4-Chlorphenyl)-4,4-dimethyl-pentan-3-on mit Trimethylsilyl-(1,2,4-triazol-1-yl)-methan umsetzt.

7. Verfahren gemäß Anspruch 1, dadurch gekennzeichnet, daß man 2,4-Dichlorbenzaldehyd mit Trimethylsilyl-(1,2,4-triazol-1-yl)-methan umsetzt.

8. Trimethylsilyl-(1,2,4-triazol-1-yl)-methan der Formel

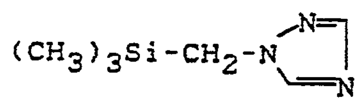 .

9. Verfahren zur Herstellung von Trimethylsilyl-(1,2,4-triazol-1-yl)-methan der Formel

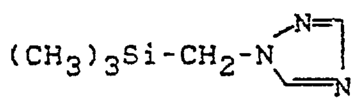 ,

dadurch gekennzeichnet, daß man Trimethyl-chlor-methyl-silan der Formel
$(CH_3)_3SiCH_2Cl$ (IV)
mit 1,2,4-Triazol der Formel

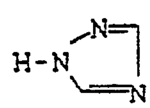 (V)

in Gegenwart eines Säurebindemittels und in Gegenwart eines Verdünnungsmittels umsetzt.

## Claims

1. Process for the preparation of β-hydroxyethylazole derivatives of the formula

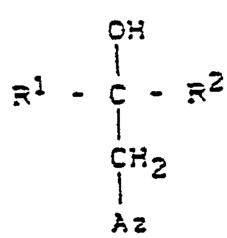 (I)

$R^1$ represents straight-chain or branched alkyl with 1 to 8 carbon atoms, straight-chain or branched halogenoalkyl with 1 to 4 carbon atoms and 1 to 5 halogen atoms, alkenyl with 2 to 8 carbon atoms, alkinyl with 2 to 8 carbon atoms, cycloalkyl which has 3 to 7 carbon atoms and is optionally substitued by alkyl with 1 or 2 carbon atoms or cycloalkenyl which has 5 to 8 carbon atoms and is optionally substituted by alkyl with 1 or 2 carbon atoms, or represents phenyl which is optionally mono-, di- or trisubstituted by identical or different substituents, possible substituents being: halogen, straight-chain or branched alkyl with 1 to

4 carbon atoms, cyclo-alkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally substituted by halogen and/or alkyl with 1 to 4 carbon atoms, or

$R^1$ represents phenylalkyl with 1 to 4 carbon atoms in the alkyl part, it being possible for the phenylalkyl radical to be mono-, di- or trisubstituted in the phenyl part by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with a to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, and/or phenylalkoxy which has 1 or 2 carbon atoms in the alkoxy part and is optionally substituted by halogen and/or alkyl with 1 to 4 carbon atoms, and

$R^2$ represents hydrogen, straight-chain or branched alkyl with 1 to 8 carbon atoms, straight-chain or branched halogenoalkyl with 1 to 4 carbon atoms and 1 to 5 halogen atoms, alkenyl with 2 to 8 carbon atoms, alkinyl with 2 to 8 carbon atoms, cycloalkyl which has 3 to 7 carbon atoms and is optionally substituted by alkyl with 1 or 2 carbon atoms or cycloalkenyl which has 5 to 8 carbon atoms and is optionally substituted by alkyl with 1 or 2 carbon atoms, or represents phenyl which is optionally mono-, di- or trisubstituted by identical or different substituents, possible substituents being: halogen, straight-chain or branched alkyl with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, and phenylalkoxy which has 1 or 2 carbon atoms in the alkoxy part and is optionally substituted by halogen and/or alkyl with 1 to 4 carbon atoms, and $R^2$ furthermore represents phenylalkyl with 1 to 4 carbon atoms in the alkyl part, it being possible for the phenylalkyl radical to be mono-, di- or trisubstituted in the phenyl part by identical or different substituents from the group comprising halogen, straight-chain or branched alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, and/or phenylalkoxy which has 1 or 2 carbon atoms in the alkoxy part and is optionally substituted by halogen and/or alkyl with 1 to 4 carbon atoms, Az represents 1,2,4-triazol-1-yl, imidazol-1-yl and pyrazol-1-yl, characterized in that carbonyl compounds of the formula

$R^1$–CO–$R^2$ (II)

in which $R^1$ and $R^2$ have the abovementioned meaning, are reacted with trimethylsilyl-azolyl-methanes of the formula

(CH3)3 Si–$CH^2$–Az (III)

in which Az has the abovementioned meaning, in the presence of a catalyst and if appropriate in the presence of a diluent at temperatures between –20°C and +130°C.

2. Process according to Claim 1, characterized in that the carbonyl compounds of the formula (II) used as starting substances are those in which

$R^1$ represents methyl, ethyl, propyl, isopropyl, n-butyl, iso-butyl, sec.-butyl or tert.-butyl or methyl, ethyl, propyl, isopropyl, n-butyl, iso-butyl, sec.-butyl or tert.-butyl each of which is substituted by 1 to 3 fluorine and/or chlorine atoms, or furthermore represents alkenyl with 2 to 6 carbon atoms, alkinyl with 2 to 6 carbon atoms, optionally methyl-substituted cyclopropyl, cyclopentyl or cyclohexyl, optionally methyl-substituted cyclopentenyl, cyclohexenyl, cycloheptenyl or cyclooctenyl, or furthermore represents phenyl which can be mono-, di- or trisubstituted by fluorine, chlorine, bromine, methyl, tert.-butyl, cyclohexyl, methoxy, methylthio, trifluoro-methyl, trifluoromethoxy, trifluoromethylthio, phenyl which is optionally substituted by fluorine, chlorine and/or methyl, phenoxy which is optionally substituted by fluorine, chlorine and/or methyl, benzyl which is optionally substituted by fluorine, chlorine and/or methyl, and/or benzyloxy which is substituted by fluorine, chlorine and/or methyl, and $R^1$ furthermore represents phenethyl which can be mono-, di- or trisubstituted in the phenyl part by fluorine, chlorine, bromine, methyl, tert.-butyl, cyclohexyl, methoxy, methylthio, trifluoromethyl, trifluoromethoxy, trifluoromethylthio, phenyl which is optionally substituted by fluorine, chlorine, and/or methyl, phenoxy which is optionally

substituted by fluorine, chlorine and/or methyl, benzyl which is optionally substituted by fluorine, chlorine and/or methyl, and/or benzyloxy which is substituted by fluorine, chlorine and/or methyl; and
$R^2$ represents hydrogen, methyl, ethyl, propyl, iso-propyl, n-butyl, iso-butyl, sec.-butyl or tert.-butyl, or methyl, ethyl, propyl, isopropyl, n-butyl, iso-butyl, sec.-butyl or tert.-butyl each of which is substituted by 1 to 3 fluorine and/or chlorine atoms, or furthermore represents alkenyl with 2 to 6 carbon atoms, optionally methyl-substituted cyclopropyl, cyclopentyl or cyclohexyl, or optionally methyl-substituted cyclopentenyl, cyclohexenyl, cycloheptenyl or cyclooctenyl, or furthermore represents phenyl which can be mono-, di- or trisubstituted by fluorine, chlorine, bromine, methyl, tert.-butyl, cyclohexyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, benzyl which is optionally substituted by fluorine, chlorine and/or methyl, and/or benzyloxy which is substituted by fluorine, chlorine and/or methyl, and
$R^2$ furthermore represents phenethyl which can be mono-, di- or trisubstituted in the phenyl part by fluorine, chlorine, bromine, methyl, tert.-butyl, cyclohexyl, methoxy, methylthio, trifluoromethyl, trifluoromethoxy, trifluoromethylthio, phenyl which is optionally substituted by fluorine, chlorine and/or methyl, phenoxy which is optoinally substituted by fluorine, chlorine and/or methyl, benzyl which is optionally substituted by fluorine, chlorine and/or methyl, and/or benzyloxy which is substituted by fluorine, chlorine and/or methyl.

3. Process according to Claim 1, characterized in that tetraalkyl-ammonium fluorides are employed as catalysts.

4. Process according to Claim 1, characterized in that the reaction is carried out at temperatures between 0°C and 80°C.

5. Process according to Claim 1, characterized in that 1 to 6 moles of trimethylsilyl-azolyl-methane of the formula (III) are employed per mole of carbonyl compound of the formula (II).

6. Process according to Claim 1, characterized in that 1-(4-chlorophenyl)-4,4-dimethyl-pentan-3-one is reacted with trimethylsilyl-(1,2,4-triazol-1-yl)-methane.

7. Process according to Claim 1, characterized in that 2,4-dichlorobenzaldehyde is reacted with trimethyl-silyl-(1,2,4-triazol-1-yl)-methane.

8. Trimethylsilyl-(1,2,4-triazol-1-yl)-methane of the formula

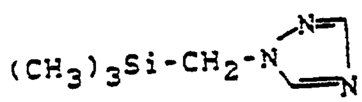

.

9. Process for the preparation of trimethylsilyl-(1,2,4-triazol-1-yl)-methane of the formula

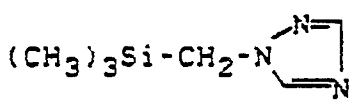

,

characterized in that trimethyl-chloromethyl-silane of the formula
$(CH_3)_3SiCH_2Cl$ (IV)
is reacted with 1,2,4-triazole of the formula

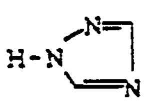

(V)

in the presence of an acid-binding agent and in the presence of a diluent.

**Revendications**

1. Procédé de préparation de dérivés du β-hydroxyéthylazole de formule:

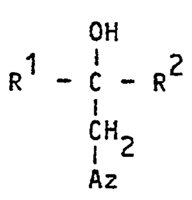

(I)

dans laquelle
$R^1$ représente un groupe alkyle, linéaire ou ramifié, ayant 1 à 8 atomes de carbone, un groupe halogéno-alkyle linéaire ou ramifié ayant 1 à 4 atomes de carbone et 1 à 5 atomes d'halogène, un groupe alcényle ayant 2 à 8 atomes de carbone, un groupe alcynyle ayant 2 à 8 atomes de carbone, un groupe cycloalkyle ayant 3 à 7 atomes de carbone (éventuellement substitué par un groupe alkyle ayant 1 ou 2 atomes de carbone), un groupe cycloalcynyle ayant 5 à 8 atomes de carbone (éventuellement substitué par un groupe alkyle ayant 1 ou 2 atomes de carbone) ou un groupe phényle éventuellement substitué 1 à 3 fois, de façon identique ou différente, et l'on peut alors citer comme substituants: de l'halogène, un groupe alkyle linéaire ou ramifié ayant 1 à 4 atomes de carbone, cycloalkyle ayant 5 à 7 atomes de carbone, alcoxy ayant 1 à 4 atomes de carbone, alkylthio ayant 1 à 4 atomes de carbone, halogéno-alkyle ayant 1 ou 2 atomes de carbone et 1 à 5 atomes d'halogène, halogéno-alcoxy ayant 1 ou 2 atomes de carbone et 1 à 5 atomes d'halogène, halogéno-alkylthio ayant 1 ou 2 atomes de carbone et 1 à 5 atomes d'halogène, un groupe phényle (éventuellement substitué par de l'halogène et/ou par un groupe alkyle ayant 1 à 4 atomes de carbone), un groupe phénoxy (éventuellement substitué par de l'halogène et/ou par un groupe alkyle ayant 1 à 4 atomes de carbone), un groupe phénylalkyle ayant 1 ou 2 atomes de carbone dans la partie alkyle (et éventuellement substitué par de l'halogène et/ou par un groupe alkyle ayant 1 à 4 atomes de carbone) ou un groupe phényl-alcoxy ayant 1 ou 2 atomes de carbone dans la partie alkyle (et éventuellement substitué par de l'halogène et/ou par un groupe alkyle ayant 1 à 4 atomes de carbone), ou bien

$R^1$ représente un groupe phénylalkyle ayant 1 à 4 atomes de carbone dans la partie alkyle, le reste phénylalkyle pouvant être substitué 1 à 3 fois, de manière identique ou différente, dans la partie phényle, par de l'halogène, par un groupe alkyle linéaire ou ramifié ayant 1 à 4 atomes de carbone, un groupe cycloalkyle ayant 5 à 7 atomes de carbone, alcoxy ayant 1 à 4 atomes de carbone, alkylthio ayant 1 à 4 atomes de carbone, halogéno-alkyle ayant 1 ou 2 atomes de carbone et 1 à 5 atomes d'halogène, halogéno-alcoxy ayant 1 ou 2 atomes de carbone et 1 à 5 atomes d'halogène, halogéno-alkylthio ayant 1 ou 2 atomes de carbone et 1 à 5 atomes d'halogène, phényle (éventuellement substitué par de l'halogène et/ou par un groupe alkyle ayant 1 à 4 atomes de carbone), phénoxy (éventuellement substitué par de l'halogène et/ou par un groupe alkyle ayant 1 à 4 atomes de carbone), phénylalkyle ayant 1 ou 2 atomes de carbone dans la partie alkyle (éventuellement substitué par de l'halogène et/ou par un groupe alkyle ayant 1 à 4 atomes de carbone) et/ou phénylalcoxy ayant 1 ou 2 atomes de carbone dans la partie alcoxy (et éventuellement substitué par de l'halogène et/ou par un groupe alkyle ayant 1 à 4 atomes de carbone), et

$R^2$ représente un atome d'hydrogène, un groupe alkyle linéaire ou ramifié ayant 1 à 8 atomes de carbone, un groupe halogéno-alkyle, linéaire ou ramifié, ayant 1 à 4 atomes de carbone et 1 à 5 atomes d'halogène, un groupe alcényle ayant 2 à 8 atomes de carbone, un groupe alcynyle ayant 2 à 8 atomes de carbone, un groupe cyclo-alkyle ayant 3 à 7 atomes de carbone (éventuellement substitué par un groupe alkyle ayant 1 ou 2 atomes de carbone), un groupe cyclo-alcényle ayant 5 à 8 atomes de carbone (éventuellement substitué par un groupe alkyle ayant 1 ou 2 atomes de carbone) ou un groupe phényle éventuellement substitué 1 à 3 fois, de manière identique ou différente, et l'on peut alors citer comme substituant: de l'halogène, un groupe alkyle linéaire ou ramifié ayant 1 à 4 atomes de carbone, alkylthio ayant 1 à 4 atomes de carbone, halogéno-alkyle ayant 1 ou 2 atomes de carbone et 1 à 5 atomes d'halogène, halogéno-alcoxy ayant 1 ou 2 atomes de carbone et 1 à 5 atomes d'halogène, halogéno-alkylthio ayant 1 ou 2 atomes de carbone et 1 à 5 atomes d'halogène, phényle (éventuellement substitué par de l'halogène et/ou par un groupe alkyle ayant 1 à 4 atomes de carbone), phénoxy (éventuellement substitué par de l'halogène et/ou par un groupe alkyle ayant 1 à 4 atomes de carbone), phénylalkyle comportant 1 ou 2 atomes de carbone dans la partie alkyle (éventuellement substitué par de l'halogène et/ou un groupe alkyle ayant 1 à 4 atomes de carbone) et phénylalcoxy ayant 1 ou 2 atomes de carbone dans la partie alcoxy (et éventuellement substitué par de l'halogène et/ou par un groupe alkyle ayant 1 à 4 atomes de carbone), et

$R^2$ représente en outre en groupe phénylalkyle ayant 1 à 4 atomes de carbone dans la partie alkyle, le reste phénylalkyle pouvant être substitué, dans la partie phényle, 1 à 3 fois de manière identique ou différente, par de l'halogène, par un groupe alkyle linéaire ou ramifié ayant 1 à 4 atomes de carbone, cycloalkyle ayant 5 à 7 atomes de carbone, alcoxy ayant 1 à 4 atomes de carbone, alkylthio ayant 1 à 4 atomes de carbone, halogéno-alkyle ayant 1 ou 2 atomes de carbone et 1 à 5 atomes d'halogène, halogéno-alcoxy ayant 1 ou 2 atomes de carbone et 1 à 5 atomes d'halogène, halogéno-alkylthio ayant 1 ou 2 atomes de carbone et 1 à 5 atomes d'halogène, phényle (éventuellement substitué par de l'halogène et/ou par un groupe phényle ayant 1 à 4 atomes de carbone), phénoxy (éventuellement substitué par de l'halogène et/ou par un groupe alkyle ayant 1 à 4 atomes de carbone), phénylalkyle ayant 1 ou 2 atomes de carbone dans la partie alkyle (et éventuellement substitué par de l'halogène et/ou par un groupe alkyle ayant 1 à 4 atomes de carbone) et/ou phénylalcoxy ayant 1 ou 2 atomes de carbone dans la partie alcoxy (et éventuellement substitué par de l'halogène et/ou par un gorupe alkyle ayant 1 à 4 atomes de carbone), et

Az représente un groupe 1,2,4-triazol-1-yle, imidazol-1-yle et pyrazol-1-yle, procédé caractérisé en ce qu'on fait réagir, en présence d'un catalyseur et éventuellement en présence d'un diluant, à des températures comprises entre −20°C et +130°C, des composés carbonylés de formule:

$R^1$–CO–$R^2$ (II)

dans laquelle $R^1$ et $R^2$ ont le sens indiqué ci-dessus, avec des triméthylsilyl-azolyl-méthanes de formule:

$(CH_3)_3Si$–$CH_2$–Az (III)

dans laquelle Az a le sens indiqué ci-dessus.

2. Procédé selon la revendication 1, caractérisé en ce qu'on utilise comme substances de départ des

composés carbonylés de formule (II), dans lesquels:
$R^1$ représente un groupe méthyle, éthyle, propyle, isopropyle, n-butyle, isobutyle, butyle secondaire, tertiobutyle, un groupe méthyle, éthyle, propyle, isopropyle, n-butyle, isobutyle, butyle secondaire ou tertiobutyle (substitués par 1 à 3 atomes de fluor et/ou de chlore), ainsi qu'un groupe alcényle ayant 2 à 6 atomes de carbone, alcynyle ayant 2 à 6 atomes de carbone, un groupe cyclopropyle, cyclopentyle ou cyclohexyle (éventuellement substitués par un groupe cyclopentényle, cyclohexényle, cycloheptényle ou cyclo-octényle (éventuellement substitués par un groupe méthyle), ainsi qu'un groupe phényle, lequel peut être substitué 1 à 3 fois par du fluor, du chlore, du brome, par un groupe méthyle, tertiobutyle, cyclohexyle, méthoxy, méthylthio, trifluorométhyle, trifluorométhoxy, trifluorométhylthio, phényle (éventuellement substitué par du fluor, du chlore et/ou un groupe méthyle), phénoxy (éventuellement substitué par du fluor, du chlore et/ou un groupe méthyle), benzyle (éventuellement substitué par du fluor, du chlore ou par un gorupe méthyle) et/ou benzyloxy (substitué par du fluor, du chlore et/ou un groupe méthyle), et
$R^1$ peut représenter en outre un groupe phénéthyle, qui peut être substitué dans la partie phényle 1 à 3 fois par du fluor, du chlore, du brome, par un groupe méthyle, tertiobutyle, cyclohexyle, méthoxy, méthylthio, trifluorométhyle, trifluorométhoxy, trifluorométhylthio, phényle (éventuellement substitué par du fluor, du chlore et/ou un groupe méthyle), phénoxy (éventuellement substitué par du fluor, du chlore et/ou un groupe méthyle), benzyle (éventuellement substitué par du fluor, du chlore et/ou un groupe méthyle) et/ou benzyloxy (éventuellement substitué par du fluor, du chlore et/ou par un groupe méthyle);
$R^2$ représente un atome d'hydrogène, un groupe méthyle, éthyle, propyle, isopropyle, n-butyle, isobutyle, butyle secondaire, tertiobutyle, un groupe méthyle, éthyle, propyle, isopropyle, n-butyle, isobutyle, butyle secondaire ou tertiobutyle (substitués 1 à 3 fois par des atomes de fluor et/ou de chlore), ainsi qu'un groupe alcényle ayant 2 à 6 atomes de carbone, un groupe cyclopropyle, cyclopentyle ou cyclohexyle (éventuellement substitués par un groupe méthyle), un groupe cyclopentényle, cyclohexényle, cycloheptényle ou cyclo-octényle (éventuellement substitués par un groupe méthyle), ainsi qu'un groupe phényle, qui peut être substitué 1 à 3 fois par du fluor, par du chlore, du brome, un groupe méthyle, tertiobutyle, cyclohexyle, méthoxy, méthylthio, trifluorométhyle, trifluorométhoxy, trifluorométhylthio, phényle (éventuellement substitué par du fluoro, par du chlore et/ou par un groupe méthyle), phénoxy (éventuellement substitué par du fluor, par du chlore et/ou par un groupe méthyle), benzyle (éventuellement substitué par du fluor, par du chlore et/ou par un groupe méthyle) et/ou benzyloxy (éventuellement substitué par du fluor, par du chlore et/ou par un groupe méthyle), et
$R^2$ représente en outre un groupe phénéthyle, qui peut être substitué dans la partie phényle 1 à 3 fois par du fluor, par du chlore, par du brome, par un groupe méthyle, tertiobutyle, cyclohexyle, methoxy, méthylthio, trifluorométhyle, trifluorométhoxy, trifluorométhylthio, phényle (éventuellement substitué par du fluor, par du chlore et/ou par un groupe méthyle), phénoxy (éventuellement substitué par du fluor, par du chlore et/ou par un groupe méthyle), benzyle (éventuellement substitué par du fluor, par du chlore et/ou un groupe méthyle) et/ou benzyloxy (éventuellement substitué par du fluoro, du chlore et/ou un groupe méthyle).

3. Procédé selon la revendication 1, caractérisé en ce qu'on utilise comme catalyseurs des fluorures de tétraalkyl-ammoniums.

4. Procédé selon la revendication 1, caractérisé en ce qu'on conduit la réaction à des températures comprises entre 0°C et 80°C.

5. Procédé selon la revendication 1, caractérisé en ce qu'on utilise, pour 1 mol du composé carbonylé de formule (II), 1 à 6 mol du triméthylsilyl-azolyl-méthane de formule (III).

6. Procédé selon la revendication 1, caractérisé en ce qu'on fait réagir la 1-(4-chlorophényl)-4,4-diméthyl-pentane-3-one avec le triméthylsilyl-(1,2,4-triazol-1-yl)-méthane.

7. Procédé selon la revendication 1, caractérisé en ce qu'on fait réagir le 2, 4,-dichlorobenzaldéhyde avec le triméthyl-silyl-(1,2,4-triazol-1-yl)-méthane.

8. Le triméthylsilyl-(1,2,4-triazol-1-yl)-méthane de formule:

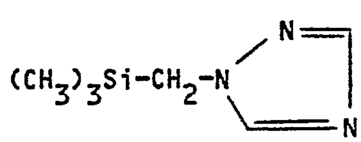

9. Procédé de préparation du triméthylsilyl-(1,2,4-triazol-1-yl)-méthane de formule:

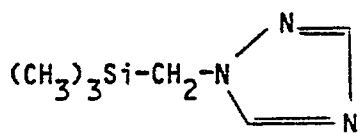

procédé caractérisé en ce qu'on fait réagir le triméthyl-chloro-méthyl-silane de formule:
$(CH3)_3SiCH_2Cl$ (IV)
avec le 1,2,4-triazole de formule:

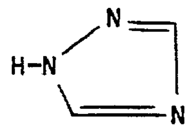

(V)

en opérant en présence d'un agent de fixation des acides et en présence d'un diluant.